# FASCICULE DE BREVET EUROPEEN

(11) **EP 0 804 449 B1**
(45) Date de publication et mention de la délivrance du brevet: **30.05.2001**
(21) Numéro de dépôt: 96939118.4
(22) Date de dépôt: 14.11.1996
(51) Int. Cl.: C07H 15/04

(54) **NOUVEAU PROCEDE DE FABRICATION DU PALATINITOL**
VERFAHREN ZUR HERSTELLUNG VON PALATINITOL
NOVEL METHOD FOR MAKING PALATINITOL

(30) Priorité: 17.11.1995 FR 9513648
(43) Date de publication de la demande: 05.11.1997
(73) Titulaire: Roquette Frères, 62136 Lestrem (FR)
(72) Inventeur: DUFLOT, Pierrick, F-62136 Richebourg (FR); FOUACHE, Catherine, F-62113 Sailly-Labourse (FR)
(74) Mandataire: Boulinguiez, Didier
(86) Numéro de dépôt international: FR9601797
(87) Numéro de publication internationale: WO9719093

(56) Documents cités:
- DE-A- 3 403 973
- DATABASE WPI Section Ch, Week 9225 Derwent Publications Ltd., London, GB; Class B03, AN 92-203078 XP002010687 & JP 04 121 198 A (SHOWA DENKO KK) , 22 Avril 1992
- JOURNAL OF THE AMERICAN CHEMICAL SOCIETY, vol. 74, 20 Février 1952, DC US, pages 1062-1064, XP002030029 WOLFROM M.L. ET AL: "Isomaltitol"

## Description

La présente invention concerne un nouveau procédé de fabrication du palatinitol.

Elle concerne plus particulièrement un procédé de fabrication du palatinitol à partir de l'isomaltose ou α-D-glucopyranosyl-(1→6)-D-glucose.

Le palatinitol est un édulcorant de masse peu calorique et peu cariogène que l'on obtient jusqu'à présent, par hydrogénation catalytique à pH neutre de l'isomaltulose ou α-D-glucopyranosyl-(1→6)-D-fructose, comme décrit dans la demande de brevet DE-A-3 403 973.

L'isomaltulose est lui même obtenu par isomérisation enzymatique, à l'aide d'une saccharose glysosyl transférase, du saccharose ou α-D-glucopyranosyl-(1-2)-β-D-fructofuranoside.

C'est donc le saccharose qui constitue la matière première d'obtention du palatinitol, mélange dans des proportions grossièrement équimoléculaires, de α-D-glucopyranosyl (1→6)-D-sorbitol (GPS ou isomaltitol) et de α-D-glucopyranosyl-(1→6)-D-mannitol (GPM).

Le palatinitol, également dénommé isomalt, est notamment commercialisé par la Société Süddeutsche Zucker AG sous le nom Palatinit®.

On pourra se référer, entre autres documents qui concernent l'obtention et les propriétés du palatinitol, à l'ouvrage "Alternative Sweeteners" édité en 1986 par LYN O'BRIEN NABORS, chapitre 11, pages 217 à 244.

Soucieuse de développer un procédé qui permette d'obtenir le palatinitol à partir d'une autre matière première que le saccharose, la Société Demanderesse a constaté que ce but pouvait être atteint par un procédé mettant en oeuvre l'isomaltose ou α-D-glucopyranosyl-(1→6)-D-glucose.

Conformément à la présente invention, on obtient le palatinitol grâce à un procédé caractérisé par le fait que
- dans une première étape, on procède à l'épimérisation de l'isomaltose dans des conditions permettant d'obtenir un mélange de α-D-glucopyranosyl-(1→6)-D-mannose et d'isomaltose,
- dans une deuxième étape, on procède à l'hydrogénation catalytique de ce mélange,
- dans une troisième étape, on procède à l'appauvrissement chromatographique en isomaltitol de ce mélange hydrogéné de manière à obtenir un mélange grossièrement équimoléculaire de α-D-glucopyranosyl-(1→6)-D-sorbitol et de α-D-glucopyranosyl-(1→6)-D-mannitol.

S'il est raisonnable d'imaginer que le palatinitol puisse être obtenu à partir de saccharose, l'homme du métier ne pouvait pas du tout s'attendre à ce que ce même palatinitol puisse être obtenu à partir d'isomaltose, lequel est obtenu à partir de glucose et donc d'amidons divers et variés.

En effet dans le premier cas, le saccharose dont la formule développée comprend un motif fructose donnera, de façon connue par isomérisation enzymatique, le cétose correspondant, soit l'isomaltulose.

Et il est connu de l'homme du métier que l'hydrogénation d'un tel cétose conduit à la formation des deux itols correspondants dans des proportions sensiblement équimoléculaires. Ainsi, le fait que la formule du saccharose s'apparente à celle du palatinitol laissait présumer du résultat.

Par contre, le procédé de l'invention ne met pas du tout en oeuvre un produit de départ dont la formule s'apparente à celle du palatinitol recherché. En effet, tant l'isomaltose que le glucose ou l'amidon ont une structure ne comportant pas de motif fructose et donc très éloignée de celle du palatinitol.

Le procédé de l'invention permet donc de s'affranchir de l'obligation d'utiliser le saccharose comme matière première de la fabrication du palatinitol puisque l'isomaltose peut être facilement obtenu à partir de glucose et donc d'amidons divers et variés, qu'ils soient issus de céréales ou de tubercules.

Un procédé d'obtention de l'isomaltose à partir du glucose ou d'un sirop de maïs est par exemple décrit dans la demande de brevet français FR-A-2 515 186.

Dans le procédé de l'invention, on préfère mettre en oeuvre de l'isomaltose cristallisé, quoique des sirops très riches en isomaltose conviennent également si l'on admet que du maltitol ou de l'isomaltotriitol peuvent être présents dans le palatinitol. Ces deux derniers composés proviennent de l'hydrogénation du maltose ou de l'isomaltotriose qui représentent les impuretés dominantes des sirops très riches en isomaltose.

Dans le procédé de l'invention, l'épimérisation de l'isomaltose peut être réalisée comme décrit dans la demande de brevet japonais JP-A-63162698 à l'aide d'un sel métallique et d'une amine mais elle est de préférence conduite de la manière qui a été décrite dans la demande de brevet japonais 63-96195 et qui consiste à faire réagir à un pH compris entre 2,5 et 4, en présence d'anhydride molybdique ou de sels de molybdène hexavalent, à une température comprise entre 90°C et 140°C, une solution aqueuse d'isomaltose.

De préférence, on emploie le molybdate d'ammonium en une proportion d'environ 0,1 à 1,5 % en poids par rapport à l'isomaltose.

De préférence encore, on procède à l'épimérisation de l'isomaltose sous forme d'une solution sucrée aqueuse contenant de 10 à 70 % d'isomaltose.

On ajuste les conditions d'épimérisation (essentiellement taux de catalyseur, durée de l'épimérisation et température de réaction), de façon à obtenir un mélange d'isomaltose et de α-D-glucopyranosyl-(1→6)-D-mannose, contenant de 10 à 40 % de ce dernier composé. Des mélanges en contenant moins de 10 % ne sont pas économiques à traiter et des mélanges en contenant plus de 40 % contiennent trop d'impuretés qui se forment dans les conditions extrêmes d'épimérisation.

On préfère travailler dans des conditions qui permettent d'obtenir 20 à 35 % de α-D-glucopyranosyl-(1→6)-D-mannose et encore plus préférentiellement, de 25 à 35 % de ce composé.

Le mélange ainsi obtenu est alors déminéralisé sur des résines échangeuses d'ions pour en ôter les sels ayant servi de catalyseur.

Dans le procédé de l'invention, l'hydrogénation du mélange épimérisé est effectuée de manière connue en soi, continue ou discontinue, sous une pression d'hydrogène de 30 à 200 bars, à une température de 80 à 150 °C en présence de catalyseur à base de nickel ou de ruthénium et à un pH proche de la neutralité. Une hydrogénation conduite à un pH inférieur à 4,0 aurait pour résultat d'hydrolyser partiellement l'isomaltose en glucose et le α-D-glucopyranosyl-(1→6)-D-mannose en glucose et mannose avec apparition de sorbitol et de mannitol. Une hydrogénation à un pH supérieur à 9 aurait pour résultat non souhaité, la formation de GPS et non de GPM à partir du α-D-glucopyranosyl-(1→6)-D-mannose. De manière générale, l'hydrogénation est poursuivie jusqu'à ce que la teneur en sucres réducteurs, mesurée par la méthode de Bertrand, devienne inférieure à 1 % et de préférence, inférieure à 0,5 %.

Après l'étape d'hydrogénation, on purifie les sirops obtenus pour en ôter le catalyseur, par filtration puis déminéralisation sur résines échangeuses d'ions, et on concentre ces sirops à une matière sèche comprise entre 10 et 70 % en vue de leur chromatographie. Ces sirops hydrogénés montrent alors une composition moyenne allant de 10 à 40 % de GPM et de 60 à 90 % de GPS. On préfère les sirops qui contiennent de 25 à 35 % de GPM et de 65 à 75 % de GPS.

Dans le procédé de l'invention, on procède ensuite à l'appauvrissement en GPS des sirops hydrogénés par voie chromatographique.

D'ordinaire, lorsque l'on fait appel à une étape chromatographique devant mener à la séparation des deux composants d'un mélange binaire, on conduit la chromatographie de manière à ce que les deux composants soient séparés de la façon la plus complète possible, c'est à dire de manière à obtenir une fraction A ne contenant que très peu de composant B et une fraction B ne contenant que très peu de composant A.

Dans le procédé de l'invention, l'appauvrissement du mélange épimérisé en GPS est au contraire conduit de manière à obtenir une fraction exclue contenant une proportion grossièrement équimoléculaire de GPS et de GPM, l'autre fraction adsorbée étant elle constituée de GPS très pur. Par grossièrement équimoléculaire, on entend de 40 à 60 % et plus préférentiellement, de 45 à 55 % d'un des deux composés par rapport à la masse totale des deux composés. Cette manière de faire présente l'avantage d'obtenir directement le palatinitol, sans devoir avoir recours à des remélanges de fractions pures de GPM et de GPS dans les proportions idoines.

La fraction chromatographique contenant le GPS excédentaire peut être commercialisée en l'état après concentration, mais on préfère en cristalliser le GPS pur que l'on sèche.

Le GPS est en effet un excellent édulcorant de masse, peu cariogène et peu calorique se présentant sous la forme d'une poudre cristalline, anhydre et blanche s'écoulant librement.

Cette étape chromatographique est menée très facilement à l'échelle industrielle par application du mélange hydrogéné sur une colonne chargée avec des résines échangeuses de cations du type polystyrène sulfoné réticulé au divinylbenzène. Ces résines, pour être adaptées à la chromatographie doivent posséder une granulométrie très fine et très homogène avantageusement comprise entre 150 et 400 microns et pour leur utilisation, sont permutées sous forme alcaline ou alcalino-terreuse. Le mélange appliqué sur la colonne est ensuite fractionné par élution à l'eau de la résine.

On constate alors de façon surprenante, que bien que le GPM et le GPS aient des structures analogues et des poids moléculaires strictement identiques, une migration beaucoup plus rapide du GPM au sein de la résine, se traduisant par un appauvrissement corrélatif en GPS du mélange soumis à la chromatographie.

Il suffit dès lors de soutirer de la résine au début du cycle d'élution, la quantité de matière juste nécessaire pour obtenir dans une proportion grossièrement stoechiométrique qui est celle du palatinitol, les composants du mélange soumis à la chromatographie.

La fraction représentant la fin du cycle d'élution contient alors une forte proportion de GPS, généralement comprise entre 80 et 95 % de la matière sèche, le reste étant essentiellement du GPM.

Cette étape de chromatographie peut être menée de façon discontinue sur une seule colonne de résine ou sur plusieurs colonnes fonctionnant de manière parallèle, mais elle est plus avantageusement conduite sur des systèmes multicolonnes branchées en boucle, fonctionnant selon le principe du lit mobile simulé. Ces systèmes ont l'avantage de tirer de meilleures performances de la résine et de fonctionner de manière continue.

D'une manière générale, pour obtenir les meilleures performances des résines de chromatographie, on préfère réaliser cette chromatographie à une température comprise entre 60 et 90°C. Comme il a déjà été dit plus haut, la fraction exclue en début du cycle d'élution, appauvrie en GPS, est avantageusement collectée en quantité telle qu'elle contienne une proportion grossièrement équimoléculaire de GPM et de GPS. La fraction adsorbée, représentant la fin du cycle d'élution et contenant essentiellement du GPS et un peu de GPM est alors concentrée puis cristallisée dans des conditions connues de l'homme de l'art pour en extraire le GPS anhydre en vue de sa commercialisation.

La fraction collectée au début du cycle d'élution et contenant en proportions grossièrement équimoléculaires le GPM et le GPS est ensuite, mais de préférence seulement, déminéralisée sur un lit mixte de résines cationique et anionique fortes puis elle est concentrée, cristallisée et séchée pour fournir une poudre commerciale de palatinitol qui est en fait un mélange en proportions grossièrement équimoléculaires d'isomaltitol anhydre et de GPM dihydrate.

La présente invention est illustrée par l'exemple qui suit et qui n'est pas limitatif, la Demanderesse n'ayant pour but que d'exposer ce qui lui paraît être l'un des meilleurs moyens de mettre en oeuvre le procédé de son invention.

### EXEMPLE

### Première étape :

On met en solution dans 36 grammes d'eau, 4 grammes d'isomaltose cristallisé ainsi que 16 mg de molybdate d'ammonium (NH₄)₆ Mo₇ O₂₄, soit 0,4 % en poids par rapport à l'isomaltose puis on ajuste le pH de cette solution à 3,5 à l'aide d'acide chlorhydrique.

On porte ensuite cette solution à 130°C durant 15 minutes.

Après refroidissement, on déminéralise cette solution sur un lit mixte de résines cationique et anionique fortes ce qui fournit un mélange épimérisé dont la résistivité est supérieure à 2.10⁶ ohms. cm.

Une chromatographie par HPLC de ce mélange épimérisé révèle la présence de 35 % de α-D-glucopyranosyl-(1→6)-D-mannose et de 65 % d'isomaltose. On observe aussi, quoiqu'à l'état de traces, la présence de glucose et de mannose.

### Deuxième étape :

On a introduit ce mélange épimérisé dans un réacteur d'hydrogénation en présence de 5 % en poids des sucres, de nickel de Raney. Après avoir mis l'appareil sous une pression d'hydrogène de 50 bars que l'on maintiendra durant toute la durée de l'hydrogénation, on chauffe son contenu à la température de 125°C. On maintient tout au cours de cette hydrogénation le pH du milieu réactionnel à 8,0 à l'aide d'une solution de bicarbonate de soude. On arrête l'hydrogénation après 8 heures, alors que la teneur en sucres réducteurs du milieu réactionnel, mesurée par la méthode de Bertrand est devenue inférieure à 0,1 %.

Le contenu du réacteur d'hydrogénation est alors filtré pour en enlever le catalyseur puis le sirop est déminéralisé sur un lit mixte de résines, comme lors de la première étape. On obtient alors un sirop parfaitement limpide et incolore dont la composition par analyse chromatographique en phase gazeuse s'avère la suivante :
isomaltitol : 64,2 %
GPM : 34,7 %

### Troisième Etape :

Dans une colonne de verre à double enveloppe thermostatée à 65°C, d'une hauteur de 2 mètres et d'un diamètre intérieur de 15 mm, on introduit 340 cm³ de la résine commercialisée sous le nom de marque PCR 732 par la Société PUROLITE. Cette résine présente les caractéristiques suivantes :
- squelette : polystyrène sulfoné réticulé au divinylbenzène
- taux de réticulation : 7 %
- granulométrie : 180 à 280 microns
- forme ionique pour l'utilisation : Ca ++

On introduit au sommet de cette colonne 2,5 cm³ du mélange hydrogéné que l'on a concentré à 10 % puis on le percole au travers de la résine en le poussant avec de l'eau à un débit de 200 cm³/heure.

Après avoir élué 169 cm³ d'eau, on commence à recueillir une fraction appauvrie en GPS, contenant le GPS et le GPM en proportions grossièrement équimoléculaires et représentant 78 cm³.

Cette fraction contenant les composants du palatinitol, à l'état dissous, montre à l'analyse chromatographique en phase gazeuse, une richesse en GPM de 49 % et une richesse en GPS de 49,5 %.

Immédiatement à la suite de cette fraction de palatinitol, on récupère une fraction de 81 cm³ constituée par un mélange très riche en GPS dont l'analyse chromatographique en phase gazeuse révèle une richesse en GPS de 92 % et de 7 % de GPM. Cette analyse révèle également des traces de sorbitol et de mannitol.

On effectue 10 fois cette étape pour obtenir une fraction de GPS adsorbée, de richesse moyenne de 91,7 % et une fraction exclue d'un mélange à parts à peu près égales de GPM et de GPS contenant 49,5 % de GPS et 49,1 % de GPM.

La fraction chromatographique adsorbée, riche en GPS a été concentrée sous vide jusqu'à une matière sèche de 75 %. En se refroidissant, elle a laissé apparaître des cristaux de GPS anhydre.

La fraction chromatographique exclue, contenant 49,1 % de GPS et 49,5 % de GPM a été concentrée de manière à amener ses deux composants à l'état de cristaux qui ont ensuite été séchés pour fournir une poudre blanche et non hygroscopique de palatinitol titrant 5,1 % d'humidité.

## Revendications

1. Procédé de fabrication du palatinitol, caractérisé par le fait que
- dans une première étape, on procède à l'épimérisation de l'isomaltose dans des conditions permettant d'obtenir un mélange de α-D-glucopyranosyl-(1→6)-D-mannose et d'isomaltose,
- dans une deuxième étape, on procède à l'hydrogénation catalytique de ce mélange,
- dans une troisième étape, on procède à l'appauvrissement chromatographique en isomaltitol de ce mélange hydrogéné de manière à obtenir un mélange grossièrement équimoléculaire de α-D-glucopyranosyl-(1→6)-D-sorbitol et de α-D-glucopyranosyl-(1→6)-D-mannitol.

2. Procédé selon la revendication 1, caractérisé par le fait que l'on procède à l'épimérisation en présence d'un sel de molybdène hexavalent.

3. Procédé selon l'une ou l'autre des revendications 1 et 2, caractérisé par le fait que l'appauvrissement chromatographique en isomaltitol du mélange hydrogéné est effectué sur des résines cationiques sous forme alcaline ou alcalino-terreuse.

4. Procédé selon la revendication 3, caractérisé par le fait que les résines cationiques sont utilisées sous forme calcium.

## Patentansprüche

1. Verfahren zur Herstellung von Palatinitol, dadurch gekennzeichnet, daß man
- in einer ersten Stufe die Epimerisierung von Isomaltose unter Bedingungen durchführt, die es ermöglichen, eine Mischung von α-D-Glucopyranosyl-(1→6)-D-Mannose und Isomaltose zu erhalten,
- in einer zweiten Stufe die katalytische Hydrierung dieser Mischung durchführt,
- in einer dritten Stufe die chromatographische Abreicherung von Isomaltose mit dieser hydrierten Mischung in der Weise durchführt, daß man eine etwa äquimolekulare Mischung von α-D-Glucopyranosyl-(1→6)-D-Sorbitol und α-D-Glucopyranosyl-(1→6)-D-Mannitol erhält.

2. Verfahren nach Anspruch 1, dadurch gekennzeichnet, daß man die Epimerisierung in Anwesenheit eines hexavalenten Molybdänsalzes durchführt.

3. Verfahren nach dem einen oder anderen der Ansprüche 1 und 2, dadurch gekennzeichnet, daß die chromatographische Abreicherung dieser hydrierten Mischung an Isomaltitol auf kationischen Harzen in der Form Alkali oder Erdalkali durchgeführt wird.

4. Verfahren nach Anspruch 3, dadurch gekennzeichnet, daß die kationischen Harze in der Form Calcium verwendet werden.

## Claims

1. Process for the preparation of palatinitol, characterized in that:
- in a first stage, the epimerization of isomaltose is carried out under conditions which allow a mixture of α-D-glucopyranosyl (1→6)-D-mannose and isomaltose to be obtained,
- in a second stage, catalytic hydrogenation is carried out on this mixture,
- in a third stage, chromatographic depletion of the isomaltose in this hydrogenated mixture is carried out so as to obtain a roughly equimolecular mixture of α-D-glucopyranosyl (1→6)-D-sorbitol and α-D-glucopyranosyl (1→6)-D-mannitol.

2. Process according to claim 1, characterized in that the epimerization is carried out in the presence of a hexavalent molybdenum salt.

3. Process according to one or other of claims 1 and 2, characterized in that chromatographic depletion of the isomaltose in the hydrogenated mixture is carried out on cationic resins in alkaline or alkaline-earth form.

4. Process according to claim 3, characterized in that the cationic resins are used in calcium form.
